(19) Europäisches Patentamt
European Patent Office
Office européen des brevets

(11) **EP 3 721 801 A1**

(12) **EUROPEAN PATENT APPLICATION**

(43) Date of publication:
**14.10.2020 Bulletin 2020/42**

(51) Int Cl.:
**A61B 5/107** (2006.01)   **A61B 5/0245** (2006.01)
**A61B 5/0402** (2006.01)   **A61B 5/00** (2006.01)

(21) Application number: **19169059.3**

(22) Date of filing: **12.04.2019**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **ETH Zurich
8092 Zurich (CH)**

(72) Inventors:
• **Dual, Seraina
8003 Zürich (CH)**
• **Schmid Daners, Marianne
8200 Schaffhausen (CH)**
• **Meboldt, Mirko
78462 Konstanz (DE)**
• **Jacob, Dominic
8400 Winterthur (CH)**
• **Pergantis, Panos
12047 Berlin (DE)**

(54) **CARDIAC DEVICE, METHOD AND COMPUTER PROGRAM PRODUCT**

(57)   A cardiac device is provided comprising a measuring electrode (3), a signal-processing unit (41,87) and a post-processing unit (43,45). The measuring electrode (3) is adapted to be positioned within the blood pool (16) of a human or an animal heart (1), in order to measure an depolarization-signal (61). The signal-processing unit (41,87) is connected to the measuring electrode (3) and is adapted to remove signal components with frequencies lower than a cut-off frequency from the measured depolarization-signal (61). The post-processing unit (43,5) is connected to the signal-processing unit (41,87) and is adapted to determine, based on the Brody effect, a measure (46) for a ventricular volume of the heart (1) based on the modified depolarization-signal (42; 5). Furthermore, a method for the determination of a measure for a ventricular volume of a heart (1) and a computer program product for performing the steps of this method are provided.

FIG. 2

EP 3 721 801 A1

FIG. 5

**Description**

TECHNICHAL FIELD

The present invention concerns a cardiac device adapted to determine a measure for a ventricular volume of a human or an animal heart, a method for determining a measure for a ventricular volume of a human or an animal heart and a computer program product for performing the steps of this method. The cardiac device can particularly be a monitoring device or a ventricular assist device (VAD) for pumping blood through a secondary intra- or extracorporeal blood circuit.

PRIOR ART

Cardiovascular disease is the leading cause of death in the developed world and the number of patients diagnosed with heart failure steadily increases. A regular or continuous monitoring of patients suffering under e.g. cardiac insufficiency at an early stage could help to recognize the development of advanced heart failure. As a consequence, suitable treatment measures could be taken such as for example a change in the medication and/or the implantation of a cardioverter-defibrillator (ICD) and/or the implantation of a ventricular assist device (VAD). Adequate timely treatment can improve the state of the heart as well as the life quality of these patients and to even prolong the lives of such patients.

For patients with advanced heart failure, the most preferred and accepted way for treatment is heart transplantation. Apart from the risk of rejection of the new heart by the body, one of the major challenges of transplantation is the insufficient supply of donor hearts. The number of heart transplants performed is insufficient for the rising demand. Thus, there is a need for alternative treatments. In this respect, the implantation of a VAD is a promising and more and more accepted method for restoring sufficient blood perfusion of patients with advanced heart failure by means of a mechanical blood pump. VADs are often used to partly or completely restore the blood perfusion of the patients for bridging the time period until transplantation. While VADs have originally been developed for bridging to transplantation, the shortage of donor hearts has shifted the focus of clinicians and VAD developers to destination therapy and bridge to recovery. Bridge to recovery aims at later explantation of the VAD, as a result of recovery of the cardiac function during mechanical circulatory support. Destination therapy denotes the use of a VAD as a permanent implant. While recovery is the most desirable outcome of VAD therapy, only few patients show enough improvement in their cardiac function permitting the removal of the VAD. Thus, destination therapy remains a valid option for the treatment of advanced heart failure.

VADs can be categorized into three pump-type generations as well as in external and internal percutaneous pump systems. First generation VADs are pneumatically or electrically actuated pulsatile volumetric pumps. Pulsatile VADs imitate the working principle of the human heart and require a pump chamber and two unidirectional-flow valves. These devices are bulky and inefficient but provide a physiological perfusion. Pulsatile VADs are nowadays used mainly for bi-ventricular support, where the balance between the left and the right ventricle poses an additional problem or in children, because the pump housing of current VADs is too big in size for the implantation. Second generation VADs are axial or centrifugal turbodynamic pumps with classical contact bearings. These devices exert a pressure on the blood by a spinning rotor in the blood stream. Modern, third generation VADs are axial or centrifugal turbodynamic pumps with a magnetically levitated rotor or with a blood-immersed bearing. Percutaneous VAD systems are intended for short-term support of cardiac function. With internal percutaneous VAD systems, such as the Impella® heart pump, a miniaturized blood pump is positioned inside of the patient's body. The small size of these pumps, however, leads to higher blood damage. In external percutaneous VAD systems, the blood pump is arranged outside of the patient's body. Second and third generation devices are small, efficient, and more reliable. Compared to pulsatile VADs, these devices, however, provide non-physiological perfusion.

A problem with VADs, and with biomedical apparatuses for pumping blood through a secondary intra- or extracorporeal blood circuit in general, concerns the controlling of the output power of the blood pump being used in these devices. Most clinically used turbodynamic and most percutaneous VADs, for example, are operated at a constant speed and the perfusion of VAD patients therefore shows only a minor adaptation to the demand. The requirements for a physiological control of blood pumps, however, are manifold and potentially conflicting. Since a controller for a turbodynamic VAD, as an example, has only one degree of freedom - the pump speed - it is not possible to meet several requirements simultaneously. It is, however, possible to reduce the set of requirements to one task: The physiological controller must prevent overpumping or underpumping, and within these limits, adapt the pump speed to meet the patient's demand. Overpumping denotes a misactuation of the VAD where the pump speed is chosen too high, which can lead to a complete emptying of the ventricle and eventually to ventricular suction. Underpumping denotes the situation where the pump speed is chosen too low, which leads to backflow through the pump and a congestion of blood before the left ventricle. This congestion increases left atrial and pulmonary venous pressures and can eventually lead to lung edema. Stagnation or backflow in the pump additionally promotes hemolysis, which is to be avoided.

Various further applications exist in which the blood of a human or an animal patient needs to be pumped through a secondary intra- or extracorporeal blood circuit. These further applications include for example the circulation of blood by means of a heart-lung machine, also referred to as cardiopulmonary bypass, or extracorporeal membrane oxygenation (ECMO), often also referred to as extracorporeal lung assist (ECLA). These applications have in common that blood from a patient is guided to a blood pump by means of an inlet duct, and from the blood pump through an outlet duct back to the patient's circulatory system. The controlling of the output power of the blood pump represents a similar challenge in all of these applications.

In the document WO 2004/073796, methods are described for regulating the blood flow rate or blood pressure in a secondary blood circuit, in order to achieve a desired blood flow rate or blood pressure in the (primary) circulatory system of a patient. For regulating the blood pump, it is suggested in this document to monitor the blood flow rate or the blood pressure in the heart or the blood vessel of the patient.

The documents WO 00/69490, WO 02/065908 and US 6,422,990 disclose VADs in which the pump speed is controlled based on measurements of external dimensions of the heart by means of sonomicrometry. The disadvantage of these devices is that a plurality of sensors has to be attached to the heart, which requires additional medical interventions for the patient.

In document WO 2012/167876, a pump for supporting the cardiac muscle is shown. For controlling the pump, a volume sensor is arranged on an inlet cannula inside the ventricle, in order to use detected volume changes for controlling the pump. The volume determination is based on the measured potential difference between a pair of electrodes, i.e. on an impedance measurement. Impedance or conductance measurements for measuring a heart volume are also disclosed in EP 2 298 375, WO 2014/085806 and in US 2003/0032853.

The provision of electrodes on the cannula of a VAD is disclosed in US 2014/0046119.

A continuous impedance measurement of ventricular volume by means of an impedance catheter for obtaining a measure for the end-diastolic volume has been proposed in Dual et al.; "R-Wave Magnitude: a Control Input for Ventricular Assist Devices"; 8th International Workshop on Biosignal Interpretation (BSI 2016), Osaka, Japan, pp. 18-21, Osaka: BSI, November 1-3, 2016.

Furthermore, unipolar electrocardiogram(ECG)-measurements using surface electrodes are proposed by Gargiulo et al. in several publications and in particular in Gargiulo et al.; "Towards true unipolar bio-potential recording: a preliminary result for ECG"; Physiol Meas. 2013 Jan;34(1):NI-7. doi: 10.1088/0967-3334/34/1/N1. Epub 2012 Dec 18.

The document WO 2007/002888 is directed to both cardiac monitoring and VADs and proposes to arrange impedance sensors within the left ventricle. Quasi-unipolar sensing is disclosed, in which the potential of an electrode arranged within the heart is referenced to the housing of a device arranged within the body of the patient, but outside of and remotely located with respect to the heart.

In US 2004/0215254 and in US 7,899,522, methods for identifying heart failure or dysfunction are proposed by means of analysing the depolarization amplitude of the intra-cardiac electromyogram (IEMG).

A biomedical apparatus for pumping blood of a human or an animal patient through a secondary intra- or extracorporeal blood circuit, in which a blood pump is controlled based on an estimate for an inner volume of the heart, is disclosed in WO 2014/173527. The estimate for the inner volume of the heart is measured by means of electromagnetical or mechanical waves.

With the devices as disclosed in the documents mentioned above, either laborious medical intervention is needed to implant the sensors in the heart or the obtained sensor data are not reliable and/or robust enough with regard to e.g. the control of a VAD or a continuous long-term monitoring. For example, the quality of estimated ventricular volumes based on impedance catheter measurements highly depends first on the calibration, which is patient specific, and second on the positioning of the electrodes for injecting an electric current and for measuring impedance. If the catheter does not remain in a constant position, recalibration is necessary. Impedance measures ventricular volume locally and is therefore limited to measuring changes in blood volume in the near field of the at least two measurement electrodes.

SUMMARY OF THE INVENTION

It is an object of the present invention to provide a cardiac device, which allows to obtain a reliable and robust measure for a ventricular volume of a human or an animal heart with reduced medical intervention.

This object is solved by a cardiac device as claimed in claim 1. Further embodiments of the cardiac device are provided in dependent claims 2 to 14. A method for the determination of a measure for a ventricular volume of a human or an animal heart, which can particularly be used in combination with a cardiac device as claimed in claim 1, is provided in claim 15. A computer program product for performing the steps of this method is provided in claims 17.

The present invention thus provides a cardiac device comprising

at least one measuring electrode adapted to be positioned within the blood pool inside or in close proximity of a human or an animal heart, in order to measure an electric depolarization-signal of the heart,

a signal-processing unit which is connected to the at least one measuring electrode and which is adapted to remove signal components with frequencies lower than a certain cut-off frequency from the measured depolarization-signal, in order to provide a modified depolarization-signal, and

a post-processing unit which is connected to the signal-processing unit and which is adapted to determine, based on the Brody effect, a measure for a ventricular volume of the heart based on the modified depolarization-signal.

The Brody effect describes the theoretical dependency of the depolarization amplitude of the intra-cardiac electromyogram (IEMG), in particular the QRS-complex and the R-wave, on the ventricular blood mass and has already been described in 1956 by D. Brody: "A theoretical analysis of intracavitary blood mass influence on the heart-lead relationship"; Circ Res. 1956 Nov; 4(6):731-8. Practical applications of the Brody effect have so far been rare due to practical problems related to the ECG or IEMG-measurement. In practical situations, the measured ECG or IEMG-signal is often impaired by the body's electric inhomogeneities i.e. lungs, by the variable distance between the measurement electrodes and the heart, and in particular by the influence of the chosen reference potential or ground (GND) on the measurement. Due to its very local nature, the Brody effect is usually not visible in the measured ECG or IEMG-signal due to adverse effects related to additional inhomogenities between the heart and the distantly located electrodes. If trying to measure the Brody effect in the surface potentials (ECG), the positioning of the electrodes as well as the position and orientation of the heart with respect to the chest wall vary from person to person inhibiting a measurement of the Brody effect in a reliable and robust manner. If trying to measure the Brody effect in the intracardiac potentials (IEMG), the positioning of the electrodes with respect to the lungs and the choice of reference potential is crucial, which should simulate a unipolar measurement as accurately as possible.

In the context of the current invention, a reliable and robust measurement of the Brody-effect became possible, however, by firstly measuring the depolarization-signal of the heart within the blood pool and inside or in close proximity of the heart using a reference potential that is as independent from the cardiac depolarization as possible and by secondly removing signal components with frequencies lower than a certain cut-off frequency from the such measured depolarization-signal.

By positioning the at least one measuring electrode within the blood pool inside or in close proximity of the heart, the measurement is carried out as close and as centrally as possible with respect to the cardiac depolarization. As a result, the individual situation, e.g. the particular anatomy of the patient, outside the heart i.e. lungs influences the measurement result to a minimal extent. Moreover, the measured depolarization-signal and particularly the signal parts thereof which are related to the Brody effect are maximal, when the at least one measuring electrode is positioned inside or in close proximity of the heart. By additionally arranging the at least one measuring electrode within the blood pool, adverse effects on the measurement result related to the local depolarization propagation over the myocardium within the individual patient are minimized.

It has been found that for enabling a reliable and robust measurement of the Brody effect, variations of the electric reference potential or ground (GND) are particularly critical and need to be avoided. Such variations can particularly be related to organic and skeletal movements of the patient. For example, movements of the heart and of surrounding organs, such as of the lungs and the thoracic diaphragm, not only during the cardiac cycle, but also during respiratory motion and/or during skeletal movements can have an influence on the chosen electric reference potential or ground (GND). These influences are pronounced, if the electric reference potential or ground (GND) is localized within the patient's body without any connection to the outside. A localization of the cardiac device and, thus, of the electric reference potential or ground (GND) completely inside the patient's body is, however, preferred, in order to allow the patient to move freely and to enable a long-term implantation of the cardiac device. It has further been found in the context of the current invention that the detrimental influences on the electric reference potential or ground (GND) can be minimized by removing signal components with frequencies lower than a certain cut-off frequency from the measured depolarization-signal. The resulting modified depolarization-signal then resembles the signal as measured by an electrode with respect of an infinite reference point, i.e. a reference point which is completely unaffected and independent of any characteristics and/or variations of the patient. As a consequence, in the context of the current invention and in this document, a measurement carried out by a single electrode in combination with the mentioned removal of low-frequency components from the measured signal is also referred to as a (quasi-)unipolar measurement by means of a (quasi-)unipolar electrode having a (quasi-)infinite reference point.

The arrangement of the at least one electrode within the blood pool means that the electrode is arranged within the blood streaming through one of the ventricles or atria of the heart or within the blood streaming through the inferior or superior vena cava, the aorta or the pulmonary artery. Thus, the at least one electrode is preferably completely surrounded by the blood streaming through the heart or a respective blood vessel.

An electrode is still considered to be positioned in close proximity of the heart, if it is e.g. not only positioned in one of the main blood vessels leading directly to or from the heart, such as the inferior or superior vena cava, the aorta or the pulmonary artery, but also in close proximity of the respective cardiac valve, i.e. one of the atrioventricular valves or one of the semilunar valves, respectively.

The depolarization of the heart which is measured by the at least one electrode can either be initiated naturally, i.e. by the heart itself, or by means of an artificial device, such as a cardiac pacemaker. With the cardiac device as described, it is usually possible to measure the depolarization in both cases and in such a way that the Brody-effect can be used for determining a measure for the ventricular volume of the heart.

In a particularly preferred embodiment, the at least one measuring electrode is adapted to be positioned within one of the ventricles or within one of the atria. An arrangement within the left ventricle is particularly preferred, because the sensitivity for measuring the depolariziation of the heart and to measure the Brody effect is optimal in this case. The expected measured depolarization amplitudes are maximal in this case. Furthermore, the left ventricular volume is most relevant to monitor the physiological and pathophysiological dynamics of the cardiovascular system.

The signal-processing unit is usually connected to the at least one measuring electrode by means of a cable. The signal-processing unit is particularly adapted to receive measurement signals of the at least one measuring electrode as an input signal. Filters and amplifiers can be included in the signal-processing unit, in order to modify the measured and received signal such that a measure for the ventricular volume can be determined optimally by the post-processing unit based on the Brody effect. The parameters of the filters and amplifiers of the signal-processing unit are preferably adjustable, in order for the user, e.g. the clinician, to be able to calibrate the cardiac device and to adjust it optimally to the patient. The signal-processing unit can particularly be formed by a printed circuit board (PCB) and a plurality of active and/or passive electronic components attached thereto, in order to carry out the desired functionality.

The measure for a ventricular volume of the heart as determined by the post-processing unit is preferably a measure for the left ventricular volume of the heart. Advantageously, the measure for a ventricular volume relates to the end-diastolic ventricular volume. Furthermore, the measure for a ventricular volume is a measure for the inner ventricular volume, i.e. the volume of the blood pool within the respective ventricle. The post-processing unit can particularly be formed by a printed circuit board (PCB) and a plurality of active and/or passive electronic components attached thereto, in order to carry out the desired functionality. The post-processing unit and the signal-processing unit are preferably arranged on the same printed circuit board (PCB).

The signal-processing unit is preferably adapted to remove the signal components from the measured depolarization-signal by low-pass filtering the measured depolarization-signal, in order to obtain a low-pass filtered depolarization-signal, and subtracting the low-pass filtered depolarization-signal from the measured depolarization-signal. Thus, a low-pass filter is preferably applied, in order to remove signal components with frequencies higher than the cut-off frequency from the measured depolarization-signal. The resulting low-pass filtered depolarization-signal is than subtracted from the measured depolarization-signal. For the subtraction, advantageously an amplifier with an inverting and a non-inverting input is used. The subtraction operation and the use of an amplifier for carrying out the subtraction operation have the advantage that a particularly high input impedance can be applied which is important with respect to the usually small biomedical signal. The removal of signal components with frequencies lower than a certain cut-off frequency from the measured depolarization-signal could, however, also been carried out by means of a respective high-pass filter.

Before reaching the signal-processing unit, the signal as directly measured by the at least one measuring electrode can optionally be pre-processed, e.g. by means of pre-filters, in order to provide a correspondingly pre-processed measured depolarization-signal to the signal-processing unit.

The cut-off frequency as applied by the signal-processing unit for removing the low-frequency signal components is preferably between 0.03 and 0.04 Hz, more preferably between 0.015 and 0.05 Hz. Experiments have shown that the choice of a cut-off frequency in the ranges as indicated is optimal to suppress unwanted signal components originating from the electric reference potential or ground (GND) on the one hand and to retain as many of the physiological signal components associated with the Brody effect on the other hand.

The cardiac device preferably comprises a plurality of measuring electrodes which are all adapted to be positioned within the blood pool inside or in close proximity of the heart, in order to measure the depolarization-signal. A larger number of measuring electrodes has the advantage of increased robustness, i.e. in the case of a failure of one measuring electrode, the measurement can be continued by another measuring electrode. Even more preferably, the cardiac device is adapted to select a subset of this plurality of measuring electrodes to measure the depolarization-signal of the heart. Thus, e.g. in a calibration and/or adaptation phase immediately after implantation, a single measuring electrode or some of the measuring electrodes can be selected, for example by the responsible clinician or automatically by the cardiac device, to carry out the depolarization-measurement. The selection can then be saved in e.g. a storage module of the cardiac device. By arranging the measuring electrodes at different positions within the heart, an optimal single measuring electrode or an optimal subset of measuring electrodes can be selected to carry out the depolarization-measurements. The plurality of measuring electrodes is advantageously applied to a common structure, e.g. an inlet cannula of a VAD.

According to a development of the invention, the electric reference potential or ground (GND) is arranged within the patient's body, but outside of the heart. The advantage of arranging the reference potential outside of the heart is that a stronger depolarization-signal can be measured and that the reference potential is less dependent on the cardiac motions. The cardiac device can comprise at least one battery which is preferably used to provide electrical energy to the signal-processing unit and/or to the post-processing unit. The at least one battery can be a primary or a secondary cell, i.e. be adapted for single use or be rechargeable, for example in the form of an accumulator. In a particularly preferred embodiment, the at least one measuring electrode is adapted to measure the depolarization-signal with respect to an electric potential defined by the at least one battery. Thus, the at least one battery is used to define the electric reference potential or ground (GND) in this case. The use of a battery and in particular the use of the middle electric potential as provided by the at least one battery leads to a reference potential which is particularly stable with respect to the depolarization-measurement.

In a different development of the invention, the electric reference potential or ground (GND) is arranged within the patient's body and even inside of the heart. By arranging the reference potential inside of the heart, influences of other organs on the measurement can be reduced.

Alternatively or in combination with the use of the at least one battery to define the reference potential, the at least one measuring electrode can also be adapted to measure the depolarization-signal with respect to an electric potential defined by one or several reference electrodes being positioned within the blood pool inside or in close proximity of the heart. Thus, a suitable combination of reference electrodes can be selected by e.g. a clinician or automatically by the cardiac device, in order to define the reference potential. The reference electrodes can particularly be selected from a plurality of measuring electrodes, meaning that some of the electrodes of the cardiac device can be selected to act as measuring electrodes and some as reference electrodes. In this way, an optimal configuration can be found to measure the Brody effect based on a plurality of electrodes provided within the heart or in close proximity thereof.

The post-processing unit is preferably adapted to identify a time sequence similar to a QRS-complex of an IEMG and to particularly detect a depolarization amplitude in the modified depolarization-signal. Furthermore, the post-processing unit is preferably adapted to determine the measure for the ventricular volume of the heart based on the peak amplitude between the zero-line or isoelectric line and a maximum absolute value of the modified depolarization-signal or based on the peak-to-peak amplitude between a minimum value and a maximum value of the modified depolarization-signal. The isoelectric line represents the base line of the electrocardiogram which is essentially flat between consecutive T- and P-or P- and Q waves. The Brody effect is particularly pronounced in the amplitudes of the R-wave. The zero-line represents the zero-or reference-potential as defined by the applied measurement system.

The cardiac device can be a monitoring device, in order to monitor the state of the heart e.g. the development of the heart during the course of chronic heart failure. For this purpose, the cardiac device can comprise an indicator device, in order to indicate a state of the heart based on the determined measure for the ventricular volume of the heart. The indicator device can also be in the form of for example an external device, such as a smart phone or a smart watch to which data from the e.g. the post-processing unit can be transmitted wirelessly by means of a transmission unit of the cardiac device arranged within the body.

The cardiac device is preferably adapted to carry out the depolarization-measurements and the determination of the measure for the ventricular volume continuously, e.g. for each cardiac cyle, or in regular time intervals. The time intervals are preferably less than 3 days and more preferably less than one day, in order to distinguish between day and night. Most preferably, the time intervals are in the range of less than 6 hours or even in the range of less than one hour.

In a particularly preferred embodiment, the cardiac device is a biomedical apparatus, in particular a VAD, for pumping blood through a secondary intra- or extracorporeal blood circuit. The biomedical apparatus in this case preferably comprises

a blood pump for pumping blood,
an inlet duct connected to the blood pump, for being inserted into a patient's circulatory system, in order to guide blood of the patient to the blood pump,
an outlet duct connected to the blood pump, for being inserted into the patient's circulatory system, in order to guide blood from the blood pump back to the patient's circulatory system, and
a controller for regulating, in particular in a closed-loop control, the power of the blood pump based on the determined measure for the ventricular volume of the heart.

The strategy to determine a measure for the ventricular volume of the heart is inspired by the Frank-Starling law describing the physiological behaviour of the stroke volume of the heart depending on the volume of the heart. According to this law, the stroke volume increases in response to an increase in the volume of blood filling the heart (the end-diastolic volume) when all other factors remain constant. Thus, by determining a measure for the ventricular volume of the heart and by using this measure for regulating the power of the blood pump, a very physiological way of regulating the blood pump is provided, in which the controller adapts the pump speed according to the patient's perfusion demand. Hence, the controller is able to adapt the cardiac output very similarly to the physiological heart. By using the measure for the ventricular volume of the heart, in order to control the blood pump, over- and underpumping are usually effectively prevented without any further measures. Effectively, if the ventricular volume is empty, the pump speed is reduced and if the ventricular volume is overly full, the pump speed is increased.

The biomedical apparatus is preferably a long-term or short-term VAD, a heart-lung machine or an extracorporeal membrane oxygenation (ECMO) apparatus. Further types of biomedical apparatuses are conceivable, such as for example a dialysis apparatus. Depending on the type of apparatus, the blood is pumped through a secondary intra- or extracorporeal blood circuit. The secondary blood circuit here refers to an additional, artificial circuit, in which blood from the (primary) circulatory system of the patient is guided to a blood pump, which pumps the blood back into the circulatory system of the patient. Within the secondary blood circuit, the blood can optionally be treated in a certain way. For example, the blood can be oxygenated (ECMO, cardiopulmonary bypass), or waste and excess water can be removed from the blood (dialysis). In these cases, in which a treatment of the blood is carried out, the secondary blood circuit is usually an extracorporeal blood circuit. In cases, in which the main function of the biomedical apparatus is to support or to replace at least a part of the function of the heart, such as in the case of a VAD, the secondary blood circuit is preferably, but not necessarily, an intracorporeal blood circuit, i.e. a blood circuit being entirely arranged within the body of the patient.

In order to optimally implement the Frank-Starling law, the controller is preferably configured to determine a measure or estimate for the end-diastolic ventricular volume (EDV) of the heart, in order to regulate the power (or pump speed) of the blood pump. The controller is particularly preferably configured to regulate the power of the blood pump based on the following linear function:

$$PW_{des}(t) = (EDV(t)\text{-}EDV_0) \cdot k_{prsw},$$

in which $PW_{des}(t)$ denotes the desired pump work per heartbeat at a certain time t and EDV(t) an estimate of the end-diastolic volume of the heart determined based on the determined measure for the ventricular volume of the heart, and in which the two parameters $EDV_0$ and $k_{prsw}$ denote the end-diastolic volume at which the desired pump work is zero and the gain of the pump work relative to the estimated EDV(t), respectively. By regulating the power according to a linear function with respect to EDV(t), the concept of the preload recruitable stroke work (PRSW) as a linear, afterload-independent model of the Frank-Starling law is implemented, as presented in: Glower DD, Spratt JA, SnowND, et al. Linearity of the frank-starling relationship in the intact heart: the concept of preload recruitable stroke work. Circ 1985; 71:994-1009. This concept has proven to be a very realistic model for the physiological regulation of the cardiac function. The control of the biomedical apparatus, and in particular of the power of the blood pump, is preferably carried out in accordance with the explanations in WO 2014/173527, the complete disclosure of which is hereby incorporated by reference.

A particularly physiological regulation of the blood pump is achieved, if $k_{prsw}$ is in the range of 0.003 J/ml to 0.02 J/ml, and in particular in the range of 0.006 J/ml to 0.012 J/ml.

In order to regulate the power of the blood pump close to the model specified by the Frank-Starling law, $EDV_0$ is preferably in the range of 10 ml to 150 ml, in particular in the range of 25 ml to 90 ml.

In a preferred embodiment, the at least one measuring electrode is arranged on the inlet duct, in particular at an open end of the inlet duct, of the biomedical apparatus. With such an arrangement of the at least one measuring electrode, the medical interventions necessary for the patient are basically the same, no matter whether the biomedical apparatus is adapted to carry out the depolarization-measurement and the measuring of the Brody effect or not. The arrangement of the at least one measuring electrode on the inlet duct and particularly at the open end of the inlet duct is particularly well suited, because the at least one measuring electrode is then usually positioned more or less centrally within the ventricle.

The cardiac device can additionally comprise at least one pressure sensor adapted to be positioned inside or in close proximity of the heart. The post-processing is then preferably adapted to determine the measure for the ventricular volume of the heart based on a combination of the modified depolarization-signal and of a value measured by the pressure sensor. A particularly robust and reliable measure for the ventricular volume can be obtained by this combination. Alternatively or in addition, the measure for the ventricular volume of the heart could also be determined based on a combination of the modified depolarization-signal and of an internal pump signal, such as the estimate of the pump flow in a VAD.

In other embodiments, the cardiac device can also be an artificial cardiac pacemaker or a cardioverter-defibrillator (ICD) comprising a controller for regulating, in particular in a closed-loop control, the heart rate of the patient based on the determined measure for the ventricular volume of the heart.

The current invention is also directed to a method for the determination of a measure for a ventricular volume of a human or an animal heart. The method can particularly be carried out by means of a cardiac device as indicated above and comprises at least the following steps:

- preferably measuring a depolarization-signal by means of at least one measuring electrode positioned within the blood pool inside or in close proximity of the heart,

- receiving, preferably by the signal-processing unit as indicated above, a depolarization-signal measured by means of at least one measuring electrode positioned within the blood pool inside or in close proximity of the heart,

- modifying, preferably by the signal-processing unit as indicated above, the depolarization-signal by removing signal components with frequencies lower than a certain cut-off frequency from the measured depolarization-signal,

- determining, preferably by the post-processing unit as indicated above, based on the Brody effect, a measure for the ventricular volume of the heart based on the modified depolarization-signal, in particular based on the peak amplitude between the zero-line or the isoelectric line and a maximum value of the modified depolarization-signal or on the peak-to-peak amplitude between a minimum value and a maximum value of the modified depolarization-signal.

According to a particularly preferred embodiment, the determined measure for the ventricular volume of the heart is used for regulating the heart rate by means of an artificial cardiac pacemaker or a cardioverter-defibrillator (ICD) or for regulating the power of a blood pump of a biomedical apparatus, in particular of a ventricular assist device (VAD), for pumping blood through a secondary intra- or extracorporeal blood circuit.

Furthermore, the current invention is directed to a computer program product directly loadable into the internal memory of a digital computer. The computer program comprises software code portions for performing the method steps of the method as indicated above, when said product is run on a computer. Thus, the software code portions of the computer program product are adapted, when being run on a computer, to carry out the above-mentioned method for the determination of a measure for a ventricular volume of a human or an animal heart. The computer program product is particularly adapted to operate and/or control the cardiac device as described above.

The computer program product is preferably stored on a storage device readable by a computer. Preferably, a non-transitory computer-readable medium is provided comprising the computer program for carrying out the method as indicated. The computer can particularly be in the form of a printed circuit board (PCB) having at least a storage module and/or processor attached thereto. The above-mentioned software code portions are in this case preferably stored in the storage module, and the processor is adapted to carry out the instructions according to these software code portions, in order to control the cardiac device.

The computer program can be realized as a computer program code element which comprises computer-implemented executable instructions to cause a processor to carry out the respective method. It can be provided in any suitable form, including source code or object code. In particular, it can be stored on a computer-readable medium or embodied in a data stream. The data stream may be accessible through a network such as the Internet.

SHORT DESCRIPTION OF THE FIGURES

Preferred embodiments of the invention are described in the following with reference to the drawings, which only serve for illustration purposes, but have no limiting effects. In the drawings it is shown:

Fig. 1    a schematic cross-sectional view of a heart with preferred positions of the measuring electrodes;
Fig. 2    a schematic view of a control unit for controlling an inventive cardiac device according to a preferred embodiment;
Fig. 3    a graph of a time sequence similar to the QRS-complex of an IEMG of a possible modified depolarization-signal as provided by a signal-processing unit of an inventive cardiac device;
Fig. 4    a schematic diagram of a signal-processing unit of an inventive cardiac device according to a preferred embodiment; and
Fig. 5    a schematic view of a cardiac device according to the invention in the form of a ventricular assist device (VAD), implanted in the heart of a patient.

DESCRIPTION OF PREFERRED EMBODIMENTS

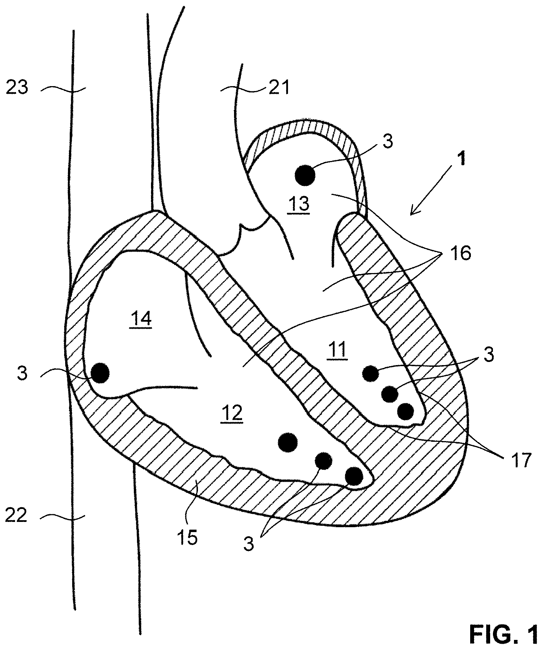

Figures 1 to 5 show preferred embodiments of the inventive cardiac device and of the inventive method for determining a measure for a ventricular volume of a human or an animal heart.

Figure 1 shows a cross-sectional view of a heart 1 which in this case is a human heart. Within the heart 1, preferred locations for positioning one or several measuring electrodes 3, in particular (quasi-)unipolar electrodes, of the inventive cardiac device are shown. The electrodes 3 for measuring the depolarization-signal are preferably arranged inside the left ventricle 11 or the right ventricle 12. In certain embodiments and/or depending on the patient to be treated, the electrodes 3 can alternatively or additionally also be positioned in the left atrium 13 or the right atrium 14. It would basically even be possible to arrange the electrode(s) 3 in the aorta 21, in the inferior vena cava 22 or in the superior vena cava 23, as long as the electrode is in close proximity of the heart, in order to be able to measure the Brody effect.

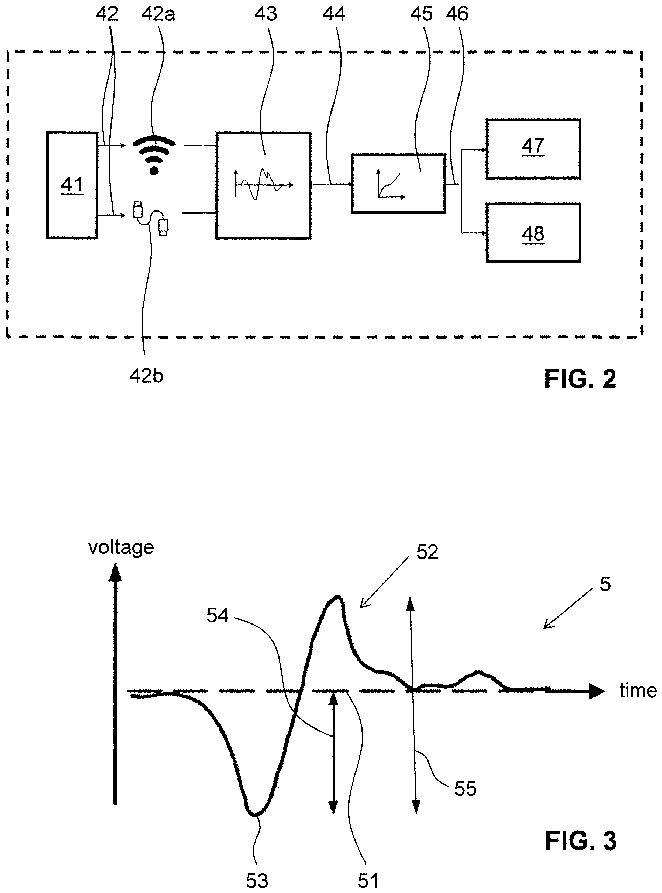

In order to avoid that the depolarization-measurement result is impaired by local (partial) depolarization effects occurring in the cardiac muscle 15, the electrodes 3 are arranged within the blood pool 16 and preferably distant from the endocardium 17. Thus, the electrodes 3 are preferably completely surrounded by the blood streaming through the atria 13, 14 and the ventricles 11, 12 of the heart 1. The arrangement of the electrodes 3 within the blood pool 16 is preferably such that they are not contacted by the endocardium 17 during the motion of the heart 1. A preferred control unit for controlling an inventive cardiac device according to a preferred embodiment is schematically shown in figure 2.

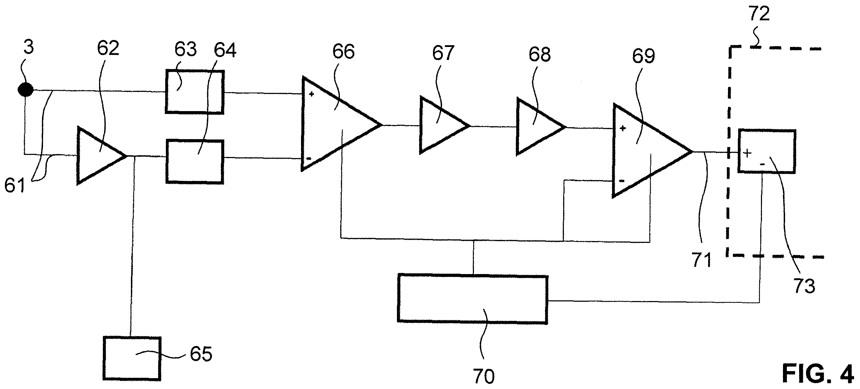

A signal measurement and processing unit 41 is connected to one or several measuring electrodes 3 located inside or in close proximity of the heart 1. The control unit and, thus, the signal measurement and processing unit 13 are as a whole located outside of the heart, but preferably within the body of a patient. The signal measurement and processing unit 41 is adapted for data acquisition. For this purpose, a depolarization-signal measured by at least one electrode 3 is received by the signal measurement and processing unit 41 and modified such, that the Brody effect can be measured from a provided modified depolarization-signal 42, i.e. that a measure for the ventricular volume of the heart 1 can be determined based on the modified depolarization-signal 42. The modification of the measured depolarization-signal as carried out by the signal measurement and processing unit 41 is explained in more detail further down with respect to figure 4. Thus, the modified depolarization-signal 42 represents the output signal of the signal measurement and processing unit 41.

The modified depolarization-signal 42 is sent via a wireless connection 42a or via a cable connection 42b from the signal measurement and processing unit 41 to a depolarization amplitude detection unit 43. The depolarization amplitude detection unit 43 is adapted to first identify a time sequence that resembles a QRS-complex of an IEMG within the received data stream of the modified depolarization-signal 42 and to then detect the depolarization amplitude within each of the identified time sequence. The detection of the depolarization amplitude as carried out by the depolarization amplitude detection unit 43 is explained in more detail further down with respect to figure 3. The detected depolarization amplitude 44 is then transmitted by the depolarization amplitude detection unit 43 in a corresponding data stream to a ventricular volume determination unit 45.

According to the Brody effect, there is a functional relationship between the amplitude 44 and the (left) ventricular end-diastolic volume of the heart 1. This functional relationship is used by the ventricular volume determination unit 45 to determine a measure 46 for the ventricular volume of the heart 1 based on the amplitude 44 as provided by the depolarization amplitude detection unit 43. Thus, the received amplitude 44 is converted by the depolarization amplitude detection unit 43 to a measure 46 for the ventricular volume, in particular for the left ventricular end-diastolic volume of the heart 1. In a preferred embodiment, a linear relationship, negative or positive depending on the electrode position, between the detected depolarization amplitude 44 and the left ventricular end-diastolic volume of the heart 1 is applied by the depolarization amplitude detection unit 43. Thus, the output signal of the depolarization amplitude detection unit 43 is the measure 46 for the ventricular volume.

The measure 46 for the ventricular volume is transmitted from the depolarization amplitude detection unit 43 to an indicator device 47 and/or to a blood pump controller 48.

The indicator device 47 serves for monitoring purposes, e.g. if the cardiac device is a monitoring device. For this purpose, the indicator device 47 which is preferably arranged inside the body of the patient can for example comprise an acoustic signal generator. In a particularly preferred embodiment, however, the indicator device 47 can be an external device to which an alarm signal and/or data concerning the state of the heart 1 can be sent from a transmission unit of the cardiac device. The transmission to the external device can be carried out wirelessly or via a cable connection. The external device is preferably located outside of the patient's body and can be formed for example by a personal computer, a laptop, a smart phone or a smart watch. The transmission unit is preferably adapted to effect data transmission upon request, e.g. from a clinician or from the patient, and/or self-triggered as a push-transmission, e.g. when detecting a special event, such as an excess of the end-diastolic volume over a certain threshold or a complete emptying of the ventricular volume, and/or after certain time intervals.

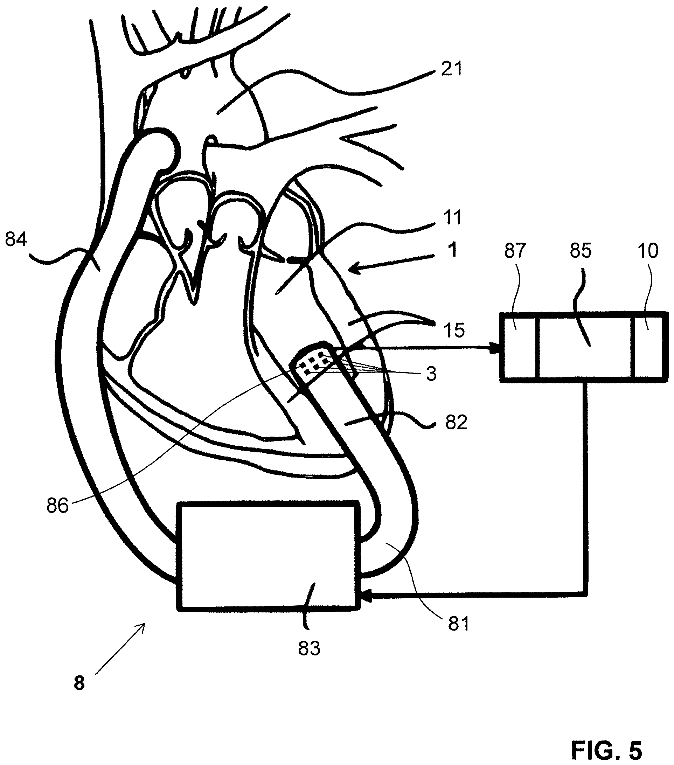

The blood pump controller 48 can be part of a biomedical apparatus, in particular a ventricular assist device (VAD), for pumping blood through a secondary intra- or extracorporeal blood circuit. In such a biomedical apparatus, the blood pump controller 48 serves to regulate the power, in particular the output power of the blood pump which is used for pumping the blood through the secondary intra- or extracorporeal blood circuit. The blood pump controller 48 can particularly be used to regulate the speed, in particular the rotational speed or the frequency of pulsation, of the blood pump. The integration of the blood pump controller 48 in a VAD will be explained in more detail further down with respect to figure 5.

Figure 3 shows a graph with a segment of a possible modified depolarization-signal 5 as provided by the signal measurement and processing unit 41. Thus, the modified depolarization-signal 5 as shown in the graph of figure 3 can particularly correspond to the modified depolarization-signal 42 as shown in figure 2.

The modified depolarization-signal 5 as shown in figure 3 contains a QRS-like complex 52 which is the result of the electrical excitation of the heart 1 during each cardiac cycle. The electrical excitation can be initiated by the heart 1 itself, i.e. naturally, or artificially by e.g. a cardiac pacemaker. The QRS-like complex 52 is represented in figure 3 with respect to an isoelectric line 51, i.e. the base line of the modified depolarization-signal 5. The QRS-like complex 52 comprises an R-wave 53 attributed to the depolarization of the left ventricle 11 of the heart 1. For determining a measure for the ventricular volume of the heart, e.g. by means of the ventricular volume determination unit 45, preferably the peak-to-peak amplitude 55 is used. The peak-to-peak amplitude 55 concerns the amplitude between a minimum value of the QRS-like complex 52, in particular the absolute minimum value of the QRS-like complex 52, and a maximum value of the QRS-like complex 52, in particular the absolute maximum value of the QRS-like complex 52. The maximum and/or minimum values of the QRS-like complex 52 are usually part of the R-wave 53. Alternatively, it is also possible to use the peak amplitude 54 for determining the measure for the ventricular volume of the heart 1. The peak amplitude 54 is measured between the isoelectric line 51 and a maximum absolute value of the QRS-like complex 52, in particular of the R-wave 53.

Figure 4 shows a schematic diagram of a possible and preferred signal-processing unit of an inventive cardiac device. The signal-processing unit as shown can for example represent a part of the signal measurement and processing unit 41 as shown in figure 2.

As shown in figure 4, an input signal 61, i.e. the measured depolarization-signal, is transmitted from the one or several electrodes 3 to a low-pass filter 62. The low-pass filter 62 according to the embodiment as shown is a 2nd-order Sallen-Key low-pass filter with 47 $\mu$F(10%) capacitors and 100 k$\Omega$(1%) resistors and having a cut-off frequency of 0.034 Hz.

From the low-pass filter 62, the filtered signal is guided, via a buffer 64, to the inverted input pin of an amplifier 66. In parallel thereto, the measured depolarization-signal is guided, via another buffer 63, from the at least one electrode 3 to the non-inverted input pin of the amplifier 66. In the current embodiment, a LM358 low power dual operational amplifier of Texas Instruments Inc. has been employed. In the amplifier 66, the low-pass filtered depolarization-signal is subtracted from the measured depolarization-signal, in order to suppress signal components which are affected by instabilities of the electric reference potential or ground (GND). The at least one electrode 3 can therefore be regarded as a unipolar electrode. Due to the use of the amplifier 66, a high input impedance of the signal-processing unit is achieved.

In cases where more than one electrode 3 is used for measuring the depolarization-signal, one or several further input channels 65 can be provided that are connected to the signal-processing unit between the low-pass filter 62 and the buffer 62.

The output of the amplifier 66 is wired to a high-pass filter 67 and to a low-pass filter 68 which are arranged in series. The high-pass filter 67 has a cut-off frequency of 0.015 Hz and serves to attenuate offsets in the depolarization-signal. The high-pass filter 67 comprises 47 $\mu$F(10%) capacitors and 220 kK2(1%) resistors. The low-pass filter 68 which is realized as 2nd-order Sallen-Key filter with 27 k$\Omega$(1%) resistors and 10 nF(5%) capacitors has a cut-off frequency of 590 Hz. The low-pass filter 68 serves to suppress high frequency noise and attenuate signal components close to and above the Nyquist-frequency of 1 kHz.

From the low-pass filter 68, the signal is then guided to the non-inverted input pin of a further amplifier 69. The inverted input pin of the amplifier 69 is connected to the ground (GND) 70. Thus, the amplifier 69 serves to amplify the depolarization-signal. From the amplifier 69, the signal is output in the form of output signal 71. The output signal 71 can particularly correspond to the modified depolarization-signal 42 or 5 as shown in figures 2 and 3, respectively.

The ground 70 is used as the ground for both the amplifier 66 and the amplifier 69.

The output signal 71 of the signal-processing unit can then be forwarded to a data acquisition unit 73 of a controller 72. The controller 72 can particularly correspond to the blood pump controller 48 as shown in figure 2.

In figure 5, an embodiment of a biomedical apparatus for pumping blood of a human or an animal patient through a secondary intra- or extracorporeal blood circuit is shown. The apparatus of this embodiment is a Ventricular Assist Device (VAD) 8 used to partially or completely replace the function of the heart 1 of a patient with heart failure.

The VAD 8 comprises a blood pump 83, which can be a pneumatically or electrically actuated pulsatile volumetric pump, or an axial or centrifugal turbodynamic pump with classical contact bearings, with a magnetically levitated rotor or with a blood-immersed bearing or a percutaneous pump. A large variety of pumps of these kinds and suited for being used in a VAD are known to the person skilled in the art.

Connected to the blood pump 83 is an inlet duct 81 having an inlet cannula 82, which has an open end being inserted into the left ventricle 11 in the region of the apex of the heart 1. The inlet cannula 82 serves to guide blood from the inside of the left ventricle 11 to the blood pump 83. Due to the pumping action of the blood pump 83, the blood is drawn through an inlet opening located at the open end of the inlet cannula 82 into the inlet duct 81 and to the blood pump 83.

In direction of the blood stream, an outlet duct 84 is connected to the blood pump 83 on the opposite side relative to the inlet duct 81. The outlet duct 84 serves to guide the blood from the blood pump 83 back to the patient's circulatory system. To this end, the outlet duct 84 is inserted into the aorta 21 of the patient.

The inlet duct 81, the blood pump 83 and the outlet duct 84 together constitute a secondary blood circuit, which is preferably located completely inside the body of the patient. The blood streaming through this secondary blood circuit originates from the left ventricle 11 and streams into the aorta 21. Within the secondary blood circuit, the blood is pumped by the blood pump 83 in the direction towards the aorta 21. Thus, the blood pump 83 supports the function of the (failed) heart 1.

The output power by which the blood is pumped into the outlet duct 84 by the blood pump 83 is regulated by a controller 85. The controller 85 can particularly correspond to the controller 72 as shown in figure 4 and/or to the blood pump controller 48 as shown in figure 2. The controller 85 is directly connected to a signal-processing unit 87 which can particularly correspond to the signal-processing unit as shown in figure 4. The signal-processing unit 87 which is connected to a plurality of electrodes 3 is preferably contained together with the controller 85 and a battery 10 in a common housing. The housing is preferably arranged within the patient's body and can be directly attached to the blood pump 83. The signal-processing unit 87 is also connected to a pressure sensor 86.

The plurality of electrodes 3 and the pressure sensor 86 are attached to the inlet cannula 82 in the region of its open end. Thus, the electrodes 3 and the pressure sensor 86 are located centrally within the left ventricle 11. In order to avoid a contact of the electrodes with the cardiac muscle 15, some or all of the electrodes 3 can be suitably shielded, e.g. by a stiff net structure, and/or be arranged in e.g. a recess provided on the inlet cannula 82. In the present embodiment, commonly used pacemaker electrodes (Biotronik 25539254 IS-1BI) have been used for the electrodes 3. The middle potential of the battery 10 is here used as the ground 70 (see figure 4) of the signal-processing unit 87.

The controller 85 is able to receive signals from the electrodes 3 and the pressure sensor 86 via the signal-processing unit 87 and is able to communicate with the blood pump 83, in order to provide a closed-loop control for the blood pump 83. The controller 85 and/or the signal-processing unit 87 can particularly be represented by an integral circuit and preferably comprise at least one data storage module. The regulation of the power of the blood pump 83 by the controller 85 is based on the determination of a measure, such as the measure 46 shown in figure 2, for the volume of the left ventricle 11 at end-diastole. In order to obtain a measure for the inner ventricular volume for regulating the power of the blood pump 83, the depolarization-signal is measured by means of the quasi-(unipolar) electrodes 3. These measurements can be combined by the controller 85 with measurements carried out by means of the pressure sensor 86.

Based on the measure for the end-diastolic volume of the heart 1, the desired stroke work ($PW_{des}$) of the blood pump 83 per heartbeat is calculated by the controller 85 according to the concept of preload recruitable stroke work (PRSW), as described in WO 2014/173527. Thus, the regulation of the power of the blood pump 83 by means of the controller 85 is based on the theory of venous return (Guyton AC, Hall JE. Textbook of Medical Physiology, 12th Edition. Saunders W B Co, 2010.) and the Frank-Starling law of the heart. This model states that the stroke work of the left ventricle 11 increases linearly with the end-diastolic volume EDV. Since the output (stroke work) of the heart 1 is proportional to the input (end-diastolic volume EDV), the PRSW can be viewed as a proportional controller of the heart 1. Hence, the controller 85 imitates the PRSW by proportionally adjusting the power of the blood pump 83 based on the determined measure for the EDV.

## REFERENCE NUMERALS

| | | | |
|---|---|---|---|
| 1 | Heart | 51 | Isoelectric line |
| 11 | Left ventricle | 52 | QRS-like complex |
| 12 | Right ventricle | 53 | R-wave |
| 13 | Left atrium | 54 | Peak amplitude |
| 14 | Right atrium | 55 | Peak-to-peak amplitude |
| 15 | Cardiac muscle | | |
| 16 | Blood pool | 61 | Input signal |
| 17 | Endocardium | 62 | Low-pass filter |
| | | 63 | Buffer |
| 21 | Aorta | 64 | Buffer |
| 22 | Inferior vena cava | 65 | Further channel |
| 23 | Superior vena cava | 66 | Amplifier |
| | | 67 | High-pass filter |
| 3 | Electrode | 68 | Low-pass filter |
| 41 | Signal measurement and processing unit | 69 | Amplifier |
| | | 70 | Ground (GND) |
| 42 | Modifed depolarization-signal | 71 | Output signal |
| | | 72 | Controller |
| 42a | Wireless connection | 73 | Data acquisition unit |
| 42b | Cable connection | 8 | Ventricular assist device (VAD) |
| 43 | Depolarization amplitude detection unit | 81 | Inlet duct |
| 44 | Amplitude | 82 | Inlet cannula |

(continued)

| 45 | Ventricular volume determination unit | 83 | Blood pump |
| --- | --- | --- | --- |
| | | 84 | Outlet duct |
| 46 | Measure for LV volume | 85 | Controller |
| 47 | Indicator device | 86 | Pressure sensor |
| 48 | Blood pump controller | 87 | Signal-processing unit |
| 5 | Modified depolarization-signal | 10 | Battery |

**Claims**

1. A cardiac device comprising

   at least one measuring electrode (3) adapted to be positioned within the blood pool (16) inside or in close proximity of a human or an animal heart (1), in order to measure an electric depolarization-signal (61) of the heart (1),
   a signal-processing unit (41, 87) which is connected to the at least one measuring electrode (3) and which is adapted to remove signal components with frequencies lower than a certain cut-off frequency from the measured depolarization-signal (61), in order to provide a modified depolarization-signal (42; 5), and
   a post-processing unit (43, 45) which is connected to the signal-processing unit (41, 87) and which is adapted to determine, based on the Brody effect, a measure (46) for a ventricular volume of the heart (1) based on the modified depolarization-signal (42; 5).

2. The cardiac device of claim 1, wherein the signal-processing unit (41, 87) is adapted to remove the signal components from the measured depolarization-signal (61) by low-pass filtering (62) the measured depolarization-signal (61), in order to obtain a low-pass filtered depolarization-signal, and subtracting the low-pass filtered depolarization-signal from the measured depolarization-signal (61).

3. The cardiac device of claim 1 or of claim 2, wherein the cut-off frequency is between 0.03 and to 0.04 Hz, in particular between 0.015 and 0.05 Hz.

4. The cardiac device of one of the preceding claims, wherein the cardiac device comprises a plurality of measuring electrodes (3) and is adapted to select a subset of these measuring electrodes (3) to measure the depolarization-signal of the heart (1).

5. The cardiac device of one of the preceding claims, wherein the cardiac device is arranged completely inside a human or animal body.

6. The cardiac device of one of the preceding claims, wherein the cardiac device comprises at least one battery (9) which is preferably used to provide electrical energy to the signal-processing unit (41, 87) and/or to the post-processing unit (43, 45), and wherein the at least one measuring electrode (3) is adapted to measure the depolarization-signal with respect to an electric potential defined by the at least one battery (9).

7. The cardiac device of one of the preceding claims, wherein the at least one measuring electrode (3) is adapted to measure the depolarization-signal with respect to an electric potential defined by one or several reference electrodes (3) being positioned within the blood pool (16) inside or in close proximity of a human or an animal heart (1).

8. The cardiac device of one of the preceding claims, wherein the post-processing unit (43, 45) is adapted to determine the measure for the ventricular volume of the heart (1) based on the peak amplitude (54) between the zero-line or isoelectric line (51) and a maximum absolute value of the modified depolarization-signal (42; 5) or based on the peak-to-peak amplitude (55) between a minimum value and a maximum value of the modified depolarization-signal (42; 5).

9. The cardiac device of one of the preceding claims, wherein the cardiac device is a monitoring device and preferably additionally comprises an indicator device (47), in order to indicate a state of the heart (1) based on the determined measure (46) for the ventricular volume of the heart (1).

10. The cardiac device of one of the preceding claims, wherein the cardiac device is an artificial cardiac pacemaker or a cardioverter-defibrillator (ICD) comprising a controller (85) for regulating the heart rate of the heart (1) based on the determined measure (46) for the ventricular volume of the heart (1).

11. The cardiac device of one of the preceding claims, wherein the cardiac device is a biomedical apparatus, in particular a ventricular assist device (VAD) (8), for pumping blood through a secondary intra- or extracorporeal blood circuit and comprises

> a blood pump (83) for pumping blood,
> an inlet duct (81) connected to the blood pump (83), for being inserted into a patient's circulatory system, in order to guide blood of the patient to the blood pump (83),
> an outlet duct (84) connected to the blood pump (83), for being inserted into the patient's circulatory system, in order to guide blood from the blood pump (83) back to the patient's circulatory system, and
> a controller (85) for regulating the power of the blood pump (83) based on the determined measure (46) for the ventricular volume of the heart (1).

12. The cardiac device of claim 11, wherein the controller (85) is configured to regulate the power of the blood pump (83) based on the following linear function:

$$PW_{des}(t) = (EDV(t)\text{-}EDV_0) \cdot k_{prsw,}$$

in which $PW_{des}(t)$ denotes the desired pump work per heartbeat at a certain time t and $EDV(t)$ an estimate for the end-diastolic volume of the heart (1) determined based on the determined measure for the ventricular volume of the heart (1), and in which the two parameters $EDV_0$ and $k_{prsw}$ denote the end-diastolic volume at which the desired pump work is zero and the gain of the pump work relative to the estimated $EDV(t)$, respectively.

13. The cadiac device of claim 11 or of claim 12, wherein the at least one measuring electrode (3) is arranged on the inlet duct (81), in particular at an open end (86) of the inlet duct (81), of the biomedical apparatus (8).

14. The cardiac device of one of the preceding claims, additionally comprising at least one pressure sensor (86) adapted to be positioned inside or in close proximity of the heart (1), wherein the post-processing unit (43, 45) is adapted to determine the measure (46) for the ventricular volume of the heart (1) based on a combination of the modified depolarization-signal (42; 5) and of a value measured by the pressure sensor (86).

15. A method for the determination of a measure for a ventricular volume of a human or an animal heart (1), in particular by means of a cardiac device as claimed in one of the preceding claims, the method comprising

> - receiving a depolarization-signal (61) measured by means of at least one measuring electrode (3) positioned within the blood pool (16) inside or in close proximity of the heart (1),
> - modifying the depolarization-signal by removing signal components with frequencies lower than a certain cut-off frequency from the measured depolarization-signal (61),
> - determining, based on the Brody effect, a measure (46) for the ventricular volume of the heart (1) based on the modified depolarization-signal (42; 5), in particular based on the peak amplitude (54) between the zero-line or isoelectric line (51) and a maximum value of the modified depolarization-signal (42; 5) or on the peak-to-peak amplitude (55) between a minimum value and a maximum value of the modified depolarization-signal (42; 5).

16. The method as claimed in claim 15, wherein the determined measure (46) for the ventricular volume of the heart (1) is used for regulating the power of a blood pump (3) of a biomedical apparatus, in particular of a ventricular assist device (VAD) (8), for pumping blood through a secondary intra- or extracorporeal blood circuit.

17. A computer program product directly loadable into the internal memory of a digital computer, comprising software code portions for performing the steps of one of claims 15 or 16, when said product is run on a computer.

**FIG. 1**

42   42a        43        44        45  46

41                                          47

                                            48

**FIG. 2**

42b

voltage

52

54

5

time

55

53   51

**FIG. 3**

FIG. 4

EP 3 721 801 A1

**FIG. 5**

Europäisches
Patentamt

European
Patent Office

Office européen
des brevets

**EUROPEAN SEARCH REPORT**

Application Number

EP 19 16 9059

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2014/173527 A1 (ETH ZUERICH [CH]) 30 October 2014 (2014-10-30) * abstract * * page 3, line 25 - line 27 * * page 7, line 24 - line 29 * * page 7, line 30 - page 8, line 14 * * page 12, line 3 - line 13 * * page 12, line 15 - line 26 * * page 13, line 15 - line 26 * * page 13, line 28 - page 14, line 14 * * page 15, line 22 - line 32 * * claim 8 * * figures 1,3,4A-B * | 1-17 | INV. A61B5/107 A61B5/0245 A61B5/0402 A61B5/00 |
| A,D | D. BRODY: "A theoretical analysis of intracavitary blood mass influence on the heart-lead relationship", CIRC RES, vol. 4, no. 6, November 1956 (1956-11), pages 731-8, XP002794401, * abstract * * sect. "Summary" * * the whole document * | 1-17 | |
| A | YUNUSEMRE OZKANLAR ET AL: "A Clinical Application of the "Brody Effect"", HEART RESEARCH - OPEN JOURNAL, vol. 2, no. 3, 28 October 2015 (2015-10-28), pages 95-99, XP055622764, DOI: 10.17140/HROJ-2-116 * abstract * * page 97 - page 98 * | 1-17 | TECHNICAL FIELDS SEARCHED (IPC) A61B |

-/--

The present search report has been drawn up for all claims

| Place of search | Date of completion of the search | Examiner |
|---|---|---|
| The Hague | 19 September 2019 | Delval, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | R Giraud ET AL: "The brody effect to detect hypovolemia in clinical practice", Critical care 2012; vol.16(suppl.1), 20 March 2012 (2012-03-20), XP055622791, DOI: 10.1186/cc10839 Retrieved from the Internet: URL:https://www.ncbi.nlm.nih.gov/pmc/articles/PMC3363650/pdf/cc10839.pdf [retrieved on 2019-09-17] * Sect. P232; page S84 * | 1-17 | |
| A | USHA B TEDROW ET AL: "Recording and interpreting unipolar electrograms to guide catheter ablation", HEART RHYTHM, vol. 8, no. 5, 1 May 2011 (2011-05-01), pages 791-796, XP028204973, ISSN: 1547-5271, DOI: 10.1016/J.HRTHM.2010.12.038 [retrieved on 2010-12-27] * sect. Unipolar recordings * * page 792 * * figure 1 * | 1-17 | |
| A | F. VANCHERI ET AL: "Relationship of Qrs amplitude to left ventricular dimensions after acute blood volume reduction in normal subjects", EUROPEAN HEART JOURNAL, vol. 10, no. 4, 1 April 1989 (1989-04-01), pages 341-345, XP055623900, GB ISSN: 0195-668X, DOI: 10.1093/oxfordjournals.eurheartj.a059491 * the whole document * | 1-17 | TECHNICAL FIELDS SEARCHED (IPC) |

The present search report has been drawn up for all claims

| Place of search | Date of completion of the search | Examiner |
|---|---|---|
| The Hague | 19 September 2019 | Delval, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 16 9059

This annex lists the patent family members relating to the patent documents cited in the above-mentioned European search report.
The members are as contained in the European Patent Office EDP file on
The European Patent Office is in no way liable for these particulars which are merely given for the purpose of information.

19-09-2019

| Patent document cited in search report | Publication date | Patent family member(s) | Publication date |
|---|---|---|---|
| WO 2014173527 A1 | 30-10-2014 | EP 2796156 A1<br>EP 2988795 A1<br>US 2016101230 A1<br>WO 2014173527 A1 | 29-10-2014<br>02-03-2016<br>14-04-2016<br>30-10-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004073796 A
- WO 0069490 A
- WO 02065908 A
- US 6422990 B
- WO 2012167876 A
- EP 2298375 A
- WO 2014085806 A
- US 20030032853 A
- US 20140046119 A
- WO 2007002888 A
- US 20040215254 A
- US 7899522 B
- WO 2014173527 A

**Non-patent literature cited in the description**

- **DUAL et al.** R-Wave Magnitude: a Control Input for Ventricular Assist Devices. *8th International Workshop on Biosignal Interpretation (BSI 2016),* 01 November 2016, 18-21
- **GARGIULO et al.** Towards true unipolar bio-potential recording: a preliminary result for ECG. *Physiol Meas.,* January 2013, vol. 34 (1), NI-7
- **D. BRODY.** A theoretical analysis of intracavitary blood mass influence on the heart-lead relationship. *Circ Res.,* November 1956, vol. 4 (6), 731-8
- **GLOWER DD ; SPRATT JA ; SNOWND et al.** Linearity of the frank-starling relationship in the intact heart: the concept of preload recruitable stroke work. *Circ,* 1985, vol. 71, 994-1009